# EUROPEAN PATENT APPLICATION

(11) **EP 3 754 662 A1**
(43) Date of publication of application: **23.12.2020**
(21) Application number: 20178935.1
(22) Date of filing: 09.06.2020
(51) Int. Cl.: G16H 10/20, G16H 20/70, G16H 40/67

(54) **SELECTION DEVICE, SELECTION METHOD, AND RECORDING MEDIUM**

(30) Priority: 21.06.2019 JP 2019115799
(71) Applicant: Unicharm Corporation, Shikokuchuo-shi, Ehime 799-0111 (JP)
(72) Inventor: Suzuki, Yuya, Kanonji-shi, Kagawa 769-1602 (JP)
(74) Representative: Dolleymores

(57) **Abstract**

A selection device (10) according to the present application includes: an acquisition unit (41) that acquires user information indicating a physical characteristic of a user, and that acquires preference information indicating a preference of the user or exercise amount information indicating an amount of exercise of the user; and a selection unit (43) that selects an absorbent article to be suggested to the user, based on the user information, and the preference information or the exercise amount information.

## Description

### CROSS-REFERENCE TO RELATED APPLICATIONS

The present application claims priority to and incorporates by reference the entire contents of Japanese Patent Application No. 2019-115799 filed in Japan on June 21, 2019.

### BACKGROUND OF THE INVENTION

### 1. Field of the Invention

The present invention relates to a selection device, a selection method, and a non-transitory computer-readable recording medium.

### 2. Description of the Related Art

Conventionally, a technology for improving the quality of life (QOL) of users by providing information on an absorbent article has been known. As an example of such technology, a technology for delivering physical advice to users, on the basis of the menstrual history of each of the users has been known (see WO 01/39584).

However, in the convention technology described above, there is much room for improving the quality of life of the user.

For example, when the user is a young woman, she may not be used to handling an absorbent article such as a sanitary product. In such a case, the user cannot select the most suitable absorbent article for herself, and while using an absorbent article, the user may feel uncomfortable or leakage may occur therefrom.

The present application has been made in view of the above, and an object of the present application is to select an absorbent article suitable for the user.

### SUMMARY OF THE INVENTION

It is an object of the present invention to at least partially solve the problems in the conventional technology. A selection device includes an acquisition unit that acquires user information indicating a physical characteristic of a user, and that acquires preference information indicating a preference of the user or exercise amount information indicating an amount of exercise of the user. The selection device includes a selection unit that selects an absorbent article to be suggested to the user, based on the user information, and the preference information or the exercise amount information.

The above and other objects, features, advantages and technical and industrial significance of this invention will be better understood by reading the following detailed description of presently preferred embodiments of the invention, when considered in connection with the accompanying drawings.

### BRIEF DESCRIPTION OF THE DRAWINGS

FIG. 1 is a diagram illustrating an example of a providing system according to an embodiment;
FIG. 2 is a diagram illustrating an example of a functional configuration of a terminal device according to the embodiment;
FIG. 3 is a diagram illustrating an example of information to be registered in a user database according to the embodiment;
FIG. 4 is a diagram illustrating an example of information to be registered in an underwear database according to the embodiment;
FIG. 5 is a diagram illustrating an example of information to be registered in an absorbent article database according to the embodiment;
FIG. 6 is a flowchart illustrating an example of a flow of a selection process executed by an information providing device according to the embodiment;
FIG. 7 is a diagram illustrating an example of suggestion information to be suggested to a parent of a user by the information providing device according to the embodiment; and
FIG. 8 is a diagram illustrating an example of a hardware configuration.

### DETAILED DESCRIPTION OF THE PREFERRED EMBODIMENTS

At least the following matters will become apparent from the description of the present specification and the accompanying drawings.

A selection device includes: an acquisition unit that acquires user information indicating physical characteristics of a user, and that acquires preference information indicating a preference of the user or exercise amount information indicating an amount of exercise of the user; and a selection unit that selects an absorbent article to be suggested to the user, on the basis of the user information, and the preference information or the exercise amount information.

For example, such a selection device selects the absorbent article that fits the user's body on the basis of the physical characteristics of the user. As a result, the selection device can prevent the absorbent article from shifting, wrinkling, and the like, and can prevent leakage and the like that occurs when the absorbent article does not fit the user's body. Moreover, as a preference for the absorbent article, the selection device selects the absorbent article on the basis of a preference of the user for appearance such as color preferred by the user and trend for illustrations, a preference of the user for the function of the absorbent article such as problems considered important by the user (wish to prevent leakage of menstrual blood, prefer an easy-handling product, prefer an inexpensive product, prefer a comfortable wearing feel, prefer to focus on the material of the product, and the like), or a preference of the user for the activities corresponding to the function of the absorbent article such as the schedule and desired activities of the user (wish to go on a trip, wish to exercise, and the like). Consequently, for example, the selection device can improve the quality of life of the user, by encouraging a young user who is not familiar with menstruation, to use the absorbent article having correct size.

Moreover, the selection device selects the absorbent article on the basis of the amount of exercise of the user. Thus, for example, the selection device can suggest the young user who exercises quite a lot, to use the absorbent article having a suitable shape or absorption function. As a result, the selection device can improve the quality of life of the user.

Moreover, the selection device acquires preference information indicating a preference of the user for the absorbent article. Thus, for example, the selection device can select the absorbent article, on the basis of the preference for the absorbent article such as a trend of design, size, texture, and the like preferred by the user. Because the selection device can encourage the young user to use the absorbent article, the selection device can improve the quality of life of the user.

Furthermore, the selection device acquires information indicating the physique of the user, as the user information. As a result, the selection device can select the absorbent article having a shape or function corresponding to the physique of the user. Thus, for example, the selection device can prevent the absorbent article from shifting or wrinkling, caused when the size of the absorbent article does not fit the physique of the user. Consequently, the selection device can prevent leakage from the absorbent article, and improve the quality of life of the user.

Still furthermore, the selection device acquires information indicating the age of the user, as the user information. As a result, the selection device can select the absorbent article corresponding to the age of the user. Thus, for example, the selection device can select the absorbent article having a shape, appearance, or function according to the age of the user. As a result, the selection device can encourage the user to use the absorbent article, and improve the quality of life of the user.

Still furthermore, the selection device further acquires time information indicating the time when the user wears the absorbent article. The selection device then selects the absorbent article to be suggested to the user, on the basis of the user information, and the preference information, the exercise amount information, or the time information. As a result, for example, the selection device can select the absorbent article that absorbs a large amount, during a period when the absorbent article cannot be replaced easily (for example, during day when there are many classes, during extracurricular activities, during night, and the like). The selection device can also select the absorbent article that does not absorb much but with less discomfort, during a period when the absorbent article can be replaced easily. As a result, the selection device can encourage the user to use the absorbent article, and improve the quality of life of the user.

Still furthermore, the selection device includes an estimation unit that estimates the circumference of the buttocks of the user, on the basis of the user information. The selection device then selects the absorbent article to be suggested to the user, on the basis of the circumference of the buttocks estimated by the estimation unit, and the preference information or the exercise amount information. The selection device may also acquire information indicating the circumference of the buttocks of the user, as the user information, and select the absorbent article to be suggested to the user, on the basis of the circumference of the buttocks of the user, and the preference information or the exercise amount information. As a result of such a process, the selection device can select the absorbent article according to the circumference of the buttocks of the user. Consequently, the selection device can select the absorbent article that fits the physique of the user.

Still furthermore, the selection device estimates underwear to be recommended to the user, on the basis of the circumference of the buttocks. Then, the selection device selects the absorbent article to be suggested to the user, on the basis of the estimated underwear. As a result of such a process, for example, the selection device can select the absorbent article having a size that does not protrude from the underwear that fits the physique of the user. Consequently, the selection device can improve the quality of life of the user.

In this example, the underwear includes underwear called sanitary shorts that improve the quality of life during menstrual period. Such sanitary shorts sometime have various functions such as a function to make the absorbent article such as a sanitary napkin used for absorbing menstrual blood fit to the body, a function to absorb menstrual blood, and a waterproof function for preventing menstrual blood from staining the other clothing, beddings, and the like. Thus, the selection device may select the absorbent article according to the function of the underwear. By selecting such an absorbent article, the selection device can further improve the quality of life during menstrual period, and improve the quality of life of the user.

Still furthermore, the selection device estimates the size of the absorbent article to be recommended on the basis of the user information, and selects the absorbent article having the estimated size. Consequently, the selection device can select the absorbent article having a size suitable for the user.

Still furthermore, the selection device estimates the function of the absorbent article to be recommended on the basis of the exercise amount information, and selects the absorbent article having the estimated function. Consequently, for example, the selection device can select the absorbent article having a function that can reduce shifting and wrinkling, such as an absorbent article with wings, to the user who does a lot of exercise.

Still furthermore, the selection device further acquires usage information indicating the absorbent article used by the user, and the usage result of the absorbent article. The selection device then selects the absorbent article to be suggested to the user, on the basis of the user information, the usage information, and the preference information or the exercise amount information. For example, when the usage information indicates that the absorbent article has not functioned properly, the selection device selects the absorbent article to be suggested to the user, from the absorbent articles excluding the absorbent article indicated in the usage information, on the basis of the user information, and the preference information or the exercise amount information. As a result, the selection device can select the absorbent article according to whether the absorbent article used by the user wrinkles, shifts, leaks, or the like. Consequently, the selection device can select the absorbent article further suitable for the user.

Still furthermore, the selection device includes a first providing unit that provides the user with information on the usage mode of the absorbent article, when the usage information indicates that the selected absorbent article and the absorbent article indicated in the usage information satisfy a predetermined condition, and when the absorbent article has not functioned properly. Consequently, for example, when leakage or the like has occurred even when the absorbent article selected from the physique and the like of the user matches with the absorbent article indicated in the usage information, the selection device provides an advice on the wearing position, the usage mode, or the like of the absorbent article. Thus, the selection device can encourage the user to use the absorbent article with the appropriate usage mode, and improve the quality of life of the user.

Still furthermore, the selection device includes a second providing unit that provides the user with information indicating a timing when replacement of the absorbent article estimated on the basis of the exercise amount information is recommended. Consequently, for example, the selection device can suggest the timing to replace the absorbent article suitable for the user, such as suggesting the user who does a lot of exercise to replace the absorbent article in advance and the like. Thus, the selection device can improve the quality of life of the user.

Still furthermore, the selection device provides the user or the other user who has a predetermined relation with the user, with information on the selected absorbent article. Consequently, for example, the selection device can provide information on the absorbent article suitable for the user, to the user, to a parent of the user, or the like. Thus, the selection device can encourage communication between the user and the parent of the user, and improve the quality of life of the user.

Hereafter, a mode (hereinafter, referred to as an "embodiment") for carrying out the selection device, the selection method, and the selection program will be described in detail with reference to the accompanying drawings. However, the selection device, the selection method, and the selection program are not limited by the embodiment. Moreover, in the following embodiment, the same reference numerals denote the same components, and repetitive description thereof will be omitted.

### Embodiment

### 1. Example of Providing System

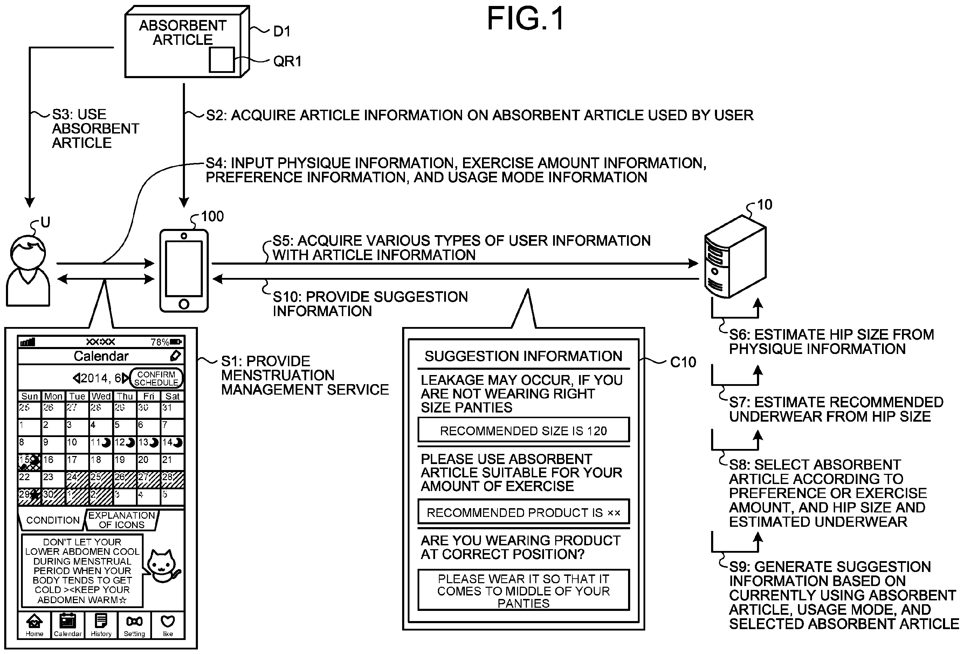

First, an example of a providing system according to an embodiment will be described with reference to FIG. 1. FIG. 1 is a diagram illustrating an example of the providing system according to the embodiment. As illustrated in FIG. 1, the providing system includes an information providing device 10 that is an example of a selection device, and a terminal device 100 used by a user U. The providing system may also include any number of the information providing devices 10 and the terminal devices 100. Moreover, the information providing device 10 may execute a process, which will be described below, with any number of the terminal devices 100.

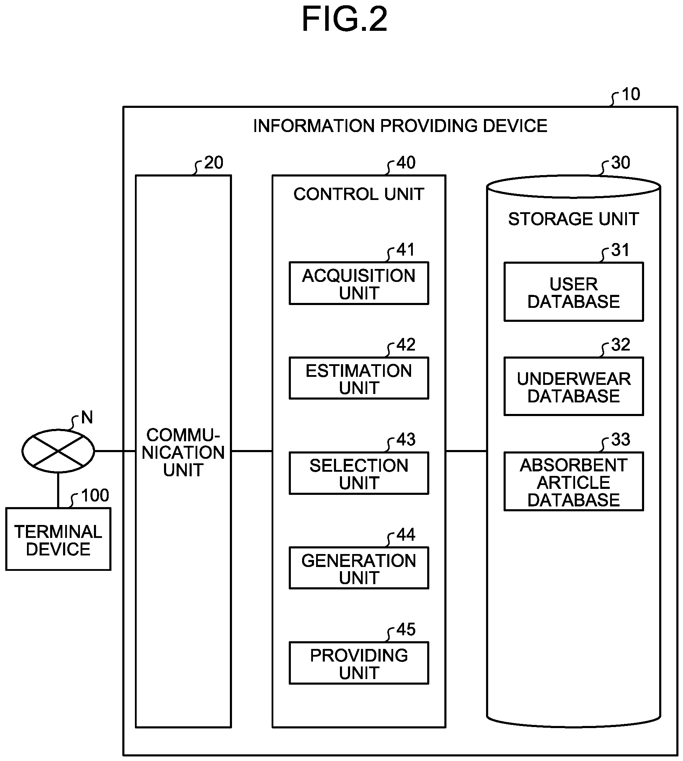

The information providing device 10 is an information processing device that performs a selection process, and for example, is implemented by a server device, a cloud system, and the like. For example, the information providing device 10 performs communication with the terminal device 100 via a network N (for example, see FIG. 2) such as a mobile communication system including Long Term Evolution (LTE), 4 Generation (G) including LTE-Advanced, which is one type of the LTE, 5G that will be standardized in future. The network N also includes various wireless communication networks such as Wifi (registered trademark) and a wireless local area network (LAN), or various wired communication networks.

The terminal device 100 is a terminal device used by the user U. The terminal device 100 is also a mobile terminal device implemented by a smart device such as a personal computer (PC), a server device, a smart television, a smartphone, or a tablet, and that can communicate with the information providing device 10 via the network N. Moreover, the terminal device 100 may include a screen such as a liquid crystal display having a touch panel function. The terminal device 100 may also receive various operations on the content distributed from the information providing device 10, such as a tap operation, a slide operation, and a scroll operation performed by the user using a finger, stylus, or the like.

### 1-1. Absorbent Article

In this example, when the user U is a female, as an example of the selection process, the information providing device 10 performs a process of selecting a female absorbent article estimated to be suitable for the user. In this example, the female absorbent articles are various sanitary products for female menstruation. As specific examples, the female absorbent articles include panty liners for vaginal discharge, sanitary napkins and tampons for menstrual bleeding, and the like. However, the embodiment is not limited thereto. For example, the absorbent article may also be an absorbent article such as an incontinence pad. The absorbent article may be worn on various underwear, and there are an underwear-type absorbent article and a sheet-type absorbent article. Moreover, the absorbent article may also be an absorbent article such as a diaper. In the following example, a process for selecting a sanitary napkin for absorbing menstrual blood is described as an example of the absorbent article. However, the selection target may also include consumer goods including an absorbent article.

Such an absorbent article differs depending on the growth, physical condition, and usage status of the wearer. For example, the menstrual blood loss of women differs according to the period of the menstrual period, the amount of exercise, night time, day time, or the like. Consequently, it is preferable to use the absorbent article having a suitable size and a function such as an absorption rate, according to a mode of menstruation of the user (whether menstrual blood loss is heavy, or whether the user does a lot of exercise), and the like.

Moreover, if the size of the absorbent article does not fit the physique of the user, or if the user does a lot of exercise, the absorbent article may wrinkle or shift. If the absorbent article does not come into close contact with the user's body, secretions such as menstrual blood and vaginal discharge may leak out from the absorbent article. Thus, it is preferable for the user to use different absorbent articles according to the user's physique, the amount of exercise, and the like.

However, some of the users are not selecting a suitable absorbent article. For example, when the user is a young person such as a junior high school student, a senior high school student, or a university student, the user may not have had her first menstrual period yet or may not be familiar with using a sanitary product because only a short period of time has passed since her first menstrual period. Consequently, there is a high possibility of leakage of secretions. As a more specific example, when the user is not familiar with the wearing feel of an absorbent article, she may wear the absorbent article on a wrong position, or carelessly wear a large absorbent article. Consequently, a gap may be formed between the absorbent article and the body of the user, and the absorbent article may be shifted or wrinkled. As a result, there is a high possibility of leakage.

Moreover, some of the young users sometimes use mother's absorbent article. Such an absorbent article often does not fit with the size of the user. Furthermore, the amount of exercise of the young user is often greater than that of her parent, due to school classes, extracurricular activities, and the like. Consequently, the sanitary product is often wrinkled or shifted, and there is a high possibility of leakage.

In this manner, if there is leakage of secretions, the secretions may stain underwear, clothing, beddings such as sheets, or the like, and the user may need to wash or hand wash the soiled items. Consequently, the user may be stressed. Moreover, the user may not be able to carry out the usual activities, due to uncomfortable feeling caused when the absorbent article is wrinkled or shifted, or by being too sensitive to leakage. Consequently, the user may be stressed. Furthermore, when the user uses absorbent article for night use during day, the absorbent article protrudes out from the underwear of the user. Consequently, the user may be stressed by worrying about how she looks.

To reduce the stress relating to the utilization of the absorbent article such as above, and to improve the quality of life of the user, it is preferable to use the absorbent article appropriately. For example, by selecting the absorbent article suitable for the user, and by using the absorbent article correctly, it is possible to prevent the absorbent article from wrinkling or shifting, and reduce the possibility of leakage. Consequently, it is possible to reduce the stress of the user, and improve the quality of life of the user.

Moreover, it is possible to reduce the possibility of leakage and improve the quality of life of the user, by allowing the user who has a little experience in using the absorbent article such as a young user, to be familiar with the correct feel of the absorbent article. Consequently, to improve the quality of life of the young user, it is considered effective to motivate the user to use such an absorbent article, in addition to suggesting the correct absorbent article and the correct usage mode.

### 1-2. Overview of Selection Process

The information providing device 10 executes the following selection process. First, the information providing device 10 acquires user information indicating the physical characteristics of the user, and acquires preference information indicating a preference of the user or exercise amount information indicating the amount of exercise of the user. Then, on the basis of the user information, and the preference information or the exercise amount information, the information providing device 10 selects the absorbent article to be suggested to the user. For example, the information providing device 10 acquires the preference information indicating a preference of the user for the absorbent article, and the user information indicating the physique of the user. Then, the information providing device 10 selects the absorbent article that meets the preference of the user, from the absorbent articles that fit the physique of the user, and suggests the user to use the selected absorbent article.

In this example, the preference for the absorbent article may be a preference for the functional attribute of the absorbent article such as a wearing feel, texture, whether the absorbent article has wings, whether the absorbent article is for night use, and the like. Moreover, the preference may also be an attribute of the appearance of the absorbent article such as a pattern and color. Furthermore, the preference for the absorbent article is a concept including various functions of the absorbent article corresponding to problems considered important by the user (wish to prevent leakage of menstrual blood, prefer a product that can be easily handled, prefer a less expensive product, prefer a comfortable wearing feel, prefer to focus on the material of the product, and the like). Still furthermore, the concept includes a preference of the user for the activities corresponding to the function of the absorbent article such as the schedule and desired activities of the user (wish to go on a trip, wish to exercise, and the like). For example, the information providing device 10 receives the preference information indicating a preference for the absorbent article, and the physique information indicating the physique of the user, from the user. In such a case, the information providing device 10 selects the absorbent article the function or the appearance of which satisfies the preference of the user, from the absorbent articles that fit the physique of the user.

Moreover, for example, when the preference information indicates that the user wishes to go out for a long period of time, in other words, when the preference information indicates the activities corresponding to the function of the absorbent article, the information providing device 10 selects the absorbent article suitable for the activities desired by the user, in other words, the absorbent article suitable for going out for a long period of time (less replacement frequency, comfortable wearing feel, and the like), or the absorbent article with a cute package, from the absorbent articles that fit the physique of the user. As a result of such a process, the information providing device 10 can motivate the young user to use the absorbent article suitable for the user, and improve the quality of life of the user. For example, various preferences for the absorbent article may also be the preference information input by the user, or may be estimated on the basis of the purchase history, activity history, and the like of the user.

Furthermore, the preference of the user such as the above may be a permanent or temporary preference. For example, the preference for appearance of the absorbent article may be a preference that continues permanently or that continues for a certain long period. The preference for the problems considered important by the user, the schedule of the user, the desired activities, or the function corresponding to the above, may be specified every time the schedule or activity is executed. It is assumed that the preference information used by the information providing device 10 indicates the various preferences.

In particular, the user who has a little experience in using the absorbent article such as a young user cannot identify the difference between various absorbent articles, and cannot determine which aspect should be taken into account to select the absorbent article. Consequently, the information providing device 10 selects the characteristics desired for the absorbent article (for example, as the absorbent article suitable for going out for a long period of time, the absorbent article having characteristics such as less replacement frequency and comfortable wearing feel), from the schedule and desired activities of the user, the attribute of appearance, and the like, as the preference of the user. As a result, the information providing device 10 can suggest the absorbent article suitable for the user, and improve the quality of life of the user. Moreover, the preference of the user such as appearance and a function of the absorbent article, and various activities that have relevance with the absorbent article described above, indicates a tendency unique to the user, with the utilization of the absorbent article. By selecting the absorbent article on the basis of such preference, the information providing device 10 can suggest the absorbent article corresponding to the activities the user wishes to perform and the like, in addition to the absorbent article the user wishes to use continuously. Consequently, the information providing device 10 can improve the quality of life of the user.

Moreover, the exercise amount information indicating the amount of exercise of the user is information indicating how much the user moves on a daily basis. For example, the information providing device 10 receives an average daily walking distance, running distance, and the like from a user, as the exercise amount information. The information providing device 10 may also receive sports played by the user such as tennis and soccer, duration of each sport, content of the extracurricular activities of the user, or the like. In such a case, the information providing device 10 selects the absorbent article having a function corresponding to the amount of exercise indicated in the exercise amount information, from the absorbent articles that fit the physique of the user. For example, the information providing device 10 may calculate an exercise amount score indicating the amount of exercise of the user, from the type, length, or the like of the sport played by the user, and select the absorbent article having a function according to the exercise amount score, from the absorbent articles that fit the physique of the user. For example, when the exercise amount score exceeds a predetermined threshold, the information providing device 10 may select the absorbent article in which the function corresponding to wrinkling or shifting is enhanced, such as an absorbent article with an adhesive tape and an absorbent article with wings. As a result of such a process, the information providing device 10 can prevent the absorbent article from shifting or wrinkling caused when the user moves, and reduce the possibility of leakage. Consequently, the information providing device 10 can improve the quality of life of the user.

Moreover, among the absorbent articles according to the physique of the user, the information providing device 10 may select the absorbent article according to the amount of exercise of the user and the absorbent article according to the preference of the user. As a result of such a process, the information providing device 10 can prevent leakage, and encourage the user to use the absorbent article that hardly leaks. Consequently, the information providing device 10 can further improve the quality of life of the user.

### 1-3. Example of Selection Process

Hereinafter, with reference to FIG. 1, an example of the selection process will be described. For example, the terminal device 100 provides a menstruation management service that manages and provides information on the menstruation of the user (step S1). For example, an application for providing the menstruation management service is installed in the terminal device 100 in advance, and the terminal device 100 manages and provides information on the menstruation of the user. For example, in the example illustrated in FIG. 1, the terminal device 100 provides the user with information on the calendar that indicates the presence of menstruation, the predicted next menstrual period, information indicating a history of poor physical condition that occurs during a menstrual period such as a stomach ache, and the like. The terminal device 100 may also provide such a menstruation management service, by cooperating with the information providing device 10 or a predetermined external server, which is not illustrated. It is assumed that the menstruation management service can provide the service with any content to the user, and the detailed description thereof will be omitted.

In this example, the terminal device 100 acquires article information on an absorbent article used by the user (step S2). Then, the user U uses an absorbent article D1 (step S3). The terminal device 100 may also acquire article information on the absorbent article before the user U uses the absorbent article D1, or acquire article information on the absorbent article after the user U has used the absorbent article D1.

For example, the terminal device 100 includes various cameras, and photographs the appearance of the absorbent article D1 according to the operation by the user U. In this example, the appearance of the absorbent article D1 may be appearance of the package in which a plurality of the absorbent articles are packed, or appearance of the individual absorbent article packed inside the package such as above. Moreover, the terminal device 100 may photograph the appearance of the absorbent article D1 through the function of an application used for providing the menstruation management service, or may photograph the appearance of the absorbent article D1 through the function of the other application.

In this example, information for specifying the absorbent article D1 such as a product name or a lot number of the absorbent article D1, in other words, a two-dimensional bar code QR1 indicating the article information is applied to the absorbent article D1. As a result, by photographing the absorbent article D1, the terminal device 100 can specify the absorbent article D1 used by the user U.

Next, the terminal device 100 receives an input of the physique information, the exercise amount information, the preference information, and the usage mode information, from the user (step S4). For example, the terminal device 100 receives various types of information from the user, through a predetermined application and the like.

In this example, the physique information is information indicating the physique of the user, and is the height and weight of the user, for example. It is assumed that the physique of the user changes according to the age of the user. Thus, the terminal device 100 may acquire information indicating the age of the user from the user, as the physique information. Moreover, as will be evident from the following description, to select the absorbent article that fits the body of the user, it is preferable to select the absorbent article having a size according to the circumference of the buttocks of the user (in other words, a hip size). Consequently, the terminal device 100 may acquire information indicating the hip size from the user, as the physique information.

Moreover, the usage mode information is information indicating the usage mode when the user U has used the absorbent article D1. For example, the terminal device 100 receives various types of information on the usage of the absorbent article D1 such as whether leakage has occurred, whether the absorbent article D1 shifts or wrinkles, and the wearing feel, when the user U has used the absorbent article D1.

Then, the information providing device 10 acquires various types of user information such as the physique information, the exercise amount information, the preference information, and the usage mode information with the article information, via the terminal device 100 (step S5). Then, the information providing device 10 estimates the hip size of the user U from the physique information (step S6). For example, the information providing device 10 may also estimate the hip size of the user U from the weight and height indicated in the physique information, or may set the hip size registered by the user U as the hip size of the user U. Moreover, the information providing device 10 may estimate the hip size of the user U from the age of the user U, and the height and weight of the user U, input as the physique information.

In this example, the information providing device 10 may select the absorbent article that fits the physique of the user directly from the hip size. However, when the usage mode of the absorbent article is taken into account, a relation with underwear such as panties may become important. For example, some women's underwear has a function for bringing a sanitary product into close contact with the user's body. When the size of such underwear does not correspond with the size of the absorbent article, there is a possibility of leakage. Moreover, when the size of underwear is not suitable, a gap and the like is formed between the sanitary product and the user' body, and there is a high possibility of leakage.

Consequently, the information providing device 10 estimates the underwear to be recommended to the user U, from the hip size (step S7). For example, the information providing device 10 has a database on the size and function of various underwear in advance, and selects the underwear suitable for the user from the hip size of the user U.

Then, the information providing device 10 selects the absorbent article according to the preference or the exercise amount, and the hip size and the estimated underwear (step S8). For example, the information providing device 10 selects the absorbent article having a size that fits the physique of the user U, from the hip size or the size of corresponding underwear. The information providing device 10 then selects the absorbent article having appearance or a function according to the preference of the user, or the absorbent article having a function according to the amount of exercise of the user, from the selected absorbent articles. For example, the information providing device 10 may also select the absorbent article having an absorption rate corresponding to the exercise amount score of the user, or the absorbent article having a function to prevent the absorbent article from shifting or wrinkling.

In this example, the information providing device 10 may provide the user U with information on the selected absorbent article. However, when the user is using the same absorbent article as the selected absorbent article, the user's impression may be spoiled. On the other hand, if there is leakage even though the user is already using the selected absorbent article, it can be considered that the wearing position or the way of wearing the absorbent article is wrong.

Consequently, the information providing device 10 generates various types of suggestion information, on the basis of the absorbent article currently being used, the usage made, and the selected absorbent article (step S9). For example, the information providing device 10 specifies the absorbent article D1 used by the user U on the basis of the article information, and specifies whether the absorbent article is shifting, wrinkling, or leaking, from the usage mode information. Then, if the absorbent article is shifting, wrinkling, or leaking, the information providing device 10 determines that the absorbent article D1 is not functioning properly.

Next, when the usage mode information indicates that the absorbent article is not functioning properly, the information providing device 10 determines whether the absorbent article selected on the basis of the physique information, the exercise amount information, the preference information, and the like matches with the absorbent article D1 indicated in the article information. When the absorbent article described above does not match with the absorbent article D1, the information providing device 10 sets the selected absorbent article as a suggestion target. In other words, the information providing device 10 selects the absorbent article to be suggested to the user, from the absorbent articles excluding the absorbent article D1. Then, the information providing device 10 generates suggestion information that suggests the user to use the selected absorbent article.

On the other hand, when the usage mode information indicates that the absorbent article D1 has functioned properly, the information providing device 10 may generate suggestion information that suggests the user to continuously use the absorbent article D1. Moreover, the information providing device 10 may generate suggestion information that suggests the user to use the absorbent article selected on the basis of the user information. For example, the information providing device 10 may also generate suggestion information that suggests the user that the absorbent article D1 is suitable, but the absorbent article selected on the basis of the user information is more suitable, when the amount of exercise is taken into consideration.

Moreover, when the absorbent article selected on the basis of the user information does not match with the absorbent article D1 indicated in the article information, the information providing device 10 may also generate suggestion information that suggests the user to use the selected absorbent article. In other words, the information providing device 10 selects the absorbent article to be suggested to the user, from the absorbent articles excluding the absorbent article D1. Then, the information providing device 10 may generate suggestion information that suggests the user to use the selected absorbent article.

In this example, when the selected absorbent article matches with the absorbent article D1, and when the absorbent article is not functioning properly, the information providing device 10 generates information on the usage mode of the absorbent article. For example, the information providing device 10 may generate suggestion information indicating the correct wearing position of the absorbent article. As a more specific example, the information providing device 10 may estimate the position where the user U is wearing the absorbent article D1, on the basis of the usage mode information, and generate suggestion information including a message to direct the user to move the absorbent article D1 to the correct position from the estimated position.

Moreover, the information providing device 10 may also generate suggestion information including information on the underwear to be recommended to the user. For example, the information providing device 10 may further reduce the possibility of leakage, by suggesting information on the underwear to be recommend from the hip size of the user at step S7, with information on the absorbent article that fits the physique of the user and that meets the preference or the amount of exercise of the user.

Then, the information providing device 10 provides the user U with the generated suggestion information, via the terminal device 100 (step S10). For example, in the example illustrated in FIG. 1, the information providing device 10 generates suggestion information C10 indicating that the size of the underwear recommended from the hip size of the user U is "120", with a message such as "Leakage may occur, if you are not wearing the right size panties". Moreover, for example, the information providing device 10 generates the suggestion information C10 indicating the absorbent article selected from the physique information and the exercise amount information of the user U, with a message such as "Please use the absorbent article suitable for your amount of exercise". Furthermore, the information providing device 10 generates the suggestion information C10 including a message such as "Are you wearing the product at the correct position?" with a message such as "Please wear it so that it comes to the middle of your panties". Then, the information providing device 10 provides the user U with the generated suggestion information C10.

As a result of such a process, the information providing device 10 can suggest, to the user, the absorbent article selected according to the physique, the amount of exercise, and the preference of the user, and provide information that encourages the user to use the correct absorbent article. As a result, the information providing device 10 can prevent leakage from the absorbent article, and improve the quality of life of the user.

### 2. Example of Functional Configuration

Hereinafter, an example of a functional configuration for implementing the selection process described above will be explained. In the following explanation, an overview of the functional configuration of the information providing device 10 is described first, and then an example of a process performed by the information providing device 10 having the functional configuration will be described.

### 2-1. Overview of Functional Configuration of Terminal Device 100

FIG. 2 is a diagram illustrating an example of a functional configuration of a terminal device according to the embodiment. As illustrated in FIG. 2, the information providing device 10 includes a communication unit 20, a storage unit 30, and a control unit 40.

The communication unit 20 is wired or wirelessly connected to the network N, and transmits and receives information with the terminal device 100. For example, the communication unit 20 is implemented by a communication circuit and the like.

For example, the storage unit 30 is implemented by a semiconductor memory element such as a random access memory (RAM) and a flash memory, or a storage device such as a hard disk and an optical disk. Various types of data for executing an output process are registered in the storage unit 30. For example, in the example illustrated in FIG. 2, a user database 31, an underwear database 32, and an absorbent article database 33 (hereinafter, may also be generally referred to as "databases 31 to 33") are registered in the storage unit 30.

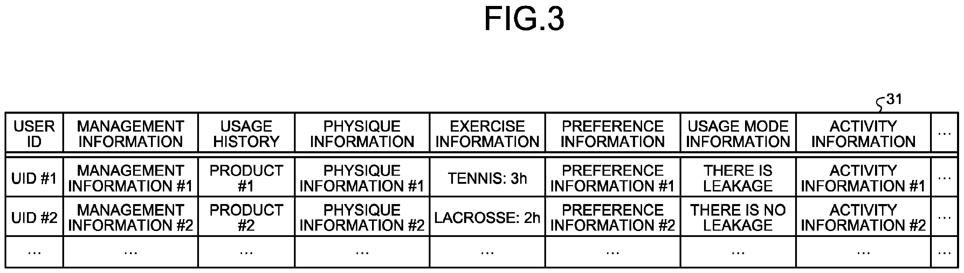

Hereinafter, with reference to FIG. 3 to FIG. 5, examples of information to be registered in the databases 31 to 33 will be described.

Information on each of the users is registered in the user database 31. For example, FIG. 3 is a diagram illustrating an example of information to be registered in the user database according to the embodiment. As illustrated in FIG. 3, information including items such as a user identifier (ID), management information, usage history, physique information, exercise information, preference information, usage mode information, and activity information is registered in the user database 31. Various types of information on the user may also be registered in the user database 31, in addition to the information illustrated in FIG. 3.

In this example, the "user ID" is an identifier for identifying a user. Moreover, the "management information" is various types of information on the menstruation management service. For example, the "management information" is the menstrual history, the next predicted menstrual period, and various types of information registered in a calendar and the like. Moreover, the "usage history" is a history of the absorbent article indicated in the article information received from the user, in other words, information indicating a history of the absorbent article used in the past by the user indicated by the corresponding user ID. Moreover, the "physique information" is information indicating the physique of the user, and for example, is information such as height and weight. Furthermore, the "exercise information" is information indicating the amount of exercise of the user, and information indicating the content of the sports played by the user and the time spent on the sports. Still furthermore, the "preference information" is information on the preference of the user. Still furthermore, the "usage mode information" is information indicating the usage mode of the absorbent article being used by the user, and is information indicating whether the absorbent article is shifting, wrinkling, leaking, or the like. Still furthermore, for example, the "activity information" is information indicating the average daily schedule of the user (for example, wake-up time, commuting time, time spent on exercise, bedtime, and the like).

For example, in the example illustrated in FIG. 3, information such as a user ID "UID #1", management information "management information #1", usage history "product #1", physique information "physique information #1", exercise information "tennis: 3h", preference information "preference information #1", usage mode information "there is leakage", and activity information "activity information #1" are registered. Such information indicates that various types of information on the menstruation management service of the user (hereinafter, referred to as a "user #1") indicated by the user ID "UID #1" is management information "management information #1". Moreover, such information indicates that the absorbent article used by the user #1 is the "product #1", and the height and weight of the user #1 are indicated in the "physique information #1". Moreover, such information indicates that the user #1 plays "tennis" for "three hours" in average, has various types of preference (for example, appearance of the absorbent article, problems considered important, and the like) indicated in the "preference information #1", and the average daily schedule of the user #1 is a schedule indicated in the "activity information #1". Moreover, such information indicates that there is leakage from the absorbent article used by the user #1.

In the example illustrated in FIG. 3, conceptual values such as the "management information #1", the "product #1", the "physique information #1", the "preference information #1", and the "activity information #1" are described. However, in reality, various types of data used in the menstruation management service; a numerical value or a character string for specifying the absorbent article; numerical values indicating the height, weight, hip size, and age; a numerical value or a character string indicating the preference; a numerical value or a character string indicating a daily schedule; and the like will be registered in the user database 31.

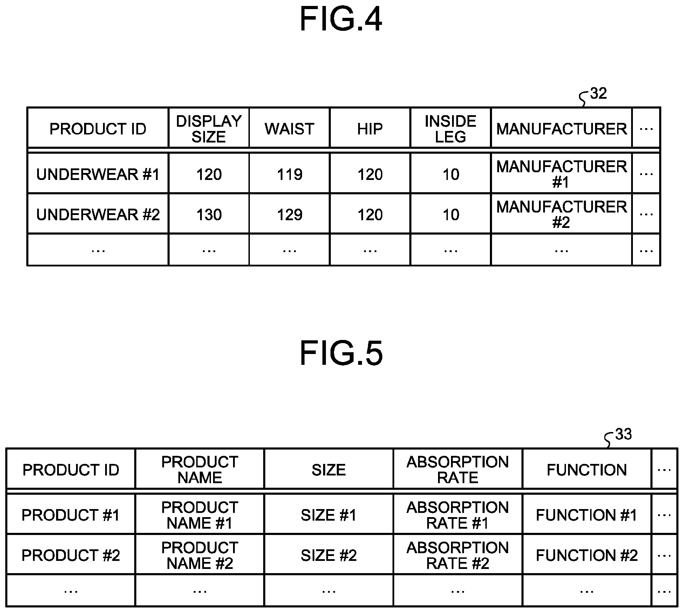

Information on underwear is registered in the underwear database 32. For example, FIG. 4 is a diagram illustrating an example of information to be registered in the underwear database according to the embodiment. As illustrated in FIG. 4, information such as an underwear ID, display size, waist, hip, inside leg, manufacturer, and the like are registered in the underwear database 32. Any information on underwear such as a function, material, pattern, and color of the underwear may also be registered in the underwear database 32, in addition to the information illustrated in FIG. 4.

The "underwear ID" is an identifier for identifying underwear. Moreover, the "display size" is size applied on the underwear, and is information indicating the corresponding hip size, for example. Moreover, the "waist" is the actual size of a portion around the waist of the underwear, the "hip" is the actual size of the hip portion of the underwear, and the "inside leg" is the actual size of the inside leg portion of the underwear. Moreover, the "manufacturer" is information indicating the manufacturer of the underwear.

For example, in the example illustrated in FIG. 4, information such as an underwear ID "underwear #1", display size "120", waist "119", hip "120", inside leg "10", and a manufacturer "manufacturer #1" are registered. Such information indicates that the display size of the underwear indicated by the underwear ID "underwear #1" is "120", the actual waist is "119" centimeters, the actual hip size is "120" centimeters, the actual inside leg is "10" centimeters, and the manufacturer is the manufacturer indicated by the "manufacturer #1".

In the example illustrated in FIG. 4, conceptual values such as the "underwear #1" and the "manufacturer #1" are described. However, in reality, a character string or a numerical value for identifying the underwear, a character string or a numerical value indicating the manufacturer, and the like will be registered in the underwear database 32.

Information on the absorbent article is registered in the absorbent article database 33. For example, FIG. 5 is a diagram illustrating an example of information to be registered in an absorbent article database according to the embodiment. As illustrated in FIG. 5, information such as a product ID, product name, size, absorption rate, function, and the like is registered in the absorbent article database 33. Information indicating the cost, stock of each store, appearance such as pattern and color, and the like may also be registered in the absorbent article database 33, in addition to the information illustrated in FIG. 5.

The "product ID" is an identifier for identifying the absorbent article. The "product name" is information indicating the name of the product. Moreover, the "size" is information indicating the size of each part of the absorbent article. Furthermore, the "absorption rate" is information indicating the amount of secretions the absorbent article can absorb. Still furthermore, the "function" is information indicating various functions on the absorbent article such as whether the absorbent article is for night use, for day use, with wings, and with an adhesive function.

For example, in the example illustrated in FIG. 5, information such as the product ID "product #1", the product name "product name #1", the size "size #1", the absorption rate "absorption rate #1", and the function "function #1" are registered in the absorbent article database 33 in an associated manner. Such information indicates that the product name of the absorbent article indicated by the product ID "product #1" is the "product name #1", and the absorbent article has the size indicated by the "size #1", the absorption rate of which is the "absorption rate #1", and has various functions indicated by the "function #1".

In the example illustrated in FIG. 5, conceptual values such as the "product name #1", the "size #1", the "absorption rate #1", and the "function #1" are described. However, in reality, a character string indicating the product name, a numerical value indicating the size, a numerical value indicating the absorption rate, a numerical value or a character string indicating various functions, and the like will be registered in the absorbent article database 33.

Returning to FIG. 2, the description will be continued. For example, the control unit 40 is an arithmetic processing device for executing various types of processing. For example, the control unit 40 is implemented when a central processing unit (CPU), a micro processing unit (MPU), or the like executes various computer programs (corresponds to an example of an output program) stored in a predetermined storage device (for example, the storage unit 30) using the RAM as a work area. Moreover, for example, the control unit 40 may also be implemented by an integrated circuit such as an application specific integrated circuit (ASIC), a field programmable gate array (FPGA), and the like.

In the example illustrated in FIG. 2, the control unit 40 includes an acquisition unit 41, an estimation unit 42, a selection unit 43, a generation unit 44, and a providing unit 45, and executes the selection process to be described below.

### 2-2. About Selection Process

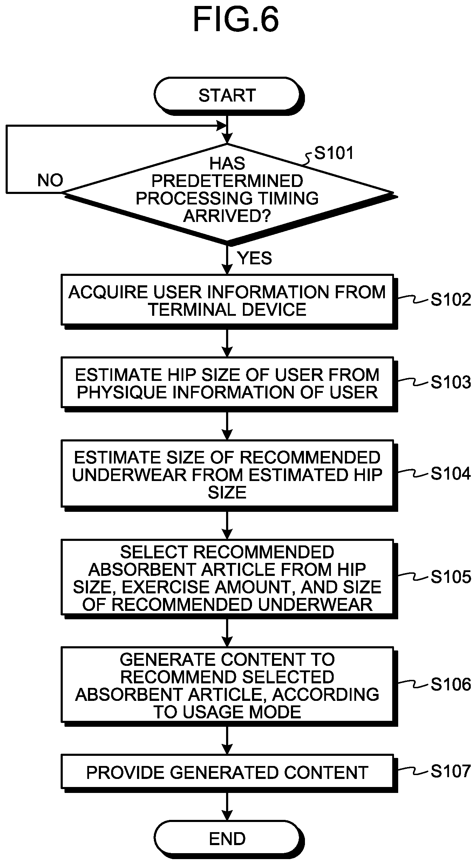

Hereinafter, an example of a flow of a selection process will be described with reference to FIG. 6. FIG. 6 is a flowchart illustrating an example of a flow of a selection process executed by the information providing device according to the embodiment. For example, the acquisition unit 41 determines whether a predetermined processing timing has arrived (step S101). When the predetermined processing timing has not arrived (No at step S101), the acquisition unit 41 waits until the predetermined processing timing. Then, when the predetermined processing timing has arrived (Yes at step S101), the acquisition unit 41 acquires various types of user information from the terminal device 100 (step S102).

For example, the acquisition unit 41 acquires the physique information indicating the physical characteristics of the user such as the height and weight, as the user information. Moreover, the acquisition unit 41 may also acquire the age of the user as a part of the physique information, or may acquire the physique information indicating the size of the circumference of the buttocks of the user. Furthermore, the acquisition unit 41 acquires the preference information indicating a preference of the user for the absorbent article or the exercise amount information indicating the amount of exercise of the user, as the user information. For example, the acquisition unit 41 acquires at least one among the preference of the user for the appearance of the absorbent article, the preference of the user for the function of the absorbent article (in other words, problems raised by the user), and the preference of the user for the activity corresponding to the function of the absorbent article (for example, a trip and the like), as the preference information. Moreover, the acquisition unit 41 may also acquire the article information indicating the absorbent article used by the user, and the usage mode information indicating the usage result. Then, the acquisition unit 41 registers the acquired various types of information in the user database 31.

In this example, the acquisition unit 41 may also acquire time information indicating the time when the user wears the absorbent article, in addition to the information described above.

Next, the estimation unit 42 estimates the hip size of the user from the physique information of the user (step S103). Then, the estimation unit 42 estimates the size of the recommended underwear from the estimated hip size (step S104). For example, the estimation unit 42 refers to the user database 31, and reads out the physique information. When the physique information includes the hip size of the user, the estimation unit 42 sets the hip size indicated in the physique information as the hip size of the user. On the other hand, when the physique information does not include the hip size, the estimation unit 42 estimates the hip size of the user from the height, weight, age, and the like indicated in the physique information. Then, the estimation unit 42 refers to the underwear database 32, and specifies the underwear corresponding to the hip size of the user.

The selection unit 43 selects the recommended absorbent article from the hip size, the amount of exercise, and the size of recommended underwear (step S105). For example, the selection unit 43 refers to the absorbent article database 33, and selects the absorbent article of a recommended size, from the hip size of the user and the recommended underwear size. The selection unit 43 may also select the absorbent article that satisfies the preference of the user, or select the absorbent article having a function such as an absorption rate or that prevents shifting, on the basis of the amount of exercise indicated in the exercise amount information of the user. In other words, the selection unit 43 selects the absorbent article to be suggested to the user, on the basis of the physique information, and the preference information or the exercise amount information.

As a more specific example, the selection unit 43 may estimate the size of the recommended absorbent article on the basis of the hip size of the user, and select the absorbent article to be suggested, from the absorbent articles with the estimated size. Moreover, the selection unit 43 may also estimate the size of the absorbent article that does not protrude out from underwear from the size of the underwear, and select the absorbent article having a size that can be fitted into the estimated size. Furthermore, when the amount of exercise indicated in the exercise amount information exceeds a predetermined threshold, the selection unit 43 may select the absorbent article that has a high absorption rate, or the absorbent article that has a function for preventing the absorbent article from wrinkling or shifting. In other words, the selection unit 43 may estimate the function of the recommended absorbent article on the basis of the exercise amount information, and select the absorbent article having the estimated function.

Moreover, when the preference information indicates a preference of the user for the appearance of the absorbent article, the selection unit 43 may select the absorbent article having color, pattern, material, or function corresponding to the preference indicated in the preference information. Moreover, for example, when the preference information indicates a preference of the user for the function of the absorbent article (in other words, problems raised by the user), the selection unit 43 may select the absorbent article having a function corresponding to such a preference (in other words, the absorbent article having a function that solves the problems of the user). Furthermore, when the preference information indicates the activities corresponding to the function of the absorbent article (for example, a trip and activities the user wishes to carry out) the selection unit 43 may select the absorbent article having a function estimated to be suitable to perform such activities. In other words, the selection unit 43 may select the absorbent article on the basis of the exercise and activities the user wishes to carry out, in addition to the actual amount of exercise, such as the amount of exercise indicated in the exercise amount information.

The selection unit 43 may also select the absorbent article according to the function of the underwear. For example, the selection unit 43 specifies the function of the underwear that is estimated as the underwear suitable for the user by the estimation unit 42. Then, the selection unit 43 may select the absorbent article having a size that does not inhibit the function of the underwear, or remove the absorbent article having a function that does not meet the function of the underwear from the selection targets. For example, when the underwear is waterproofed to accommodate leakage, the selection unit 43 may select the absorbent article having a fixing function such as wings, instead of the absorbent article having an adhesive function such as an adhesive tape.

Moreover, the selection unit 43 may select the absorbent article on the basis of the usage mode information. For example, when the usage mode information indicates that the absorbent article has not functioned properly, the selection unit 43 may select the absorbent article to be suggested to the user, from the absorbent articles excluding the absorbent article indicated in the article information, on the basis of the user information, and the preference information or the exercise amount information.

Furthermore, the selection unit 43 may also select the absorbent article to be suggested to the user, on the basis of the time information indicating the time when the user uses the absorbent article. For example, when the time information indicates that the user uses the absorbent article during day, the selection unit 43 may select the absorbent article for day use. When the time information indicates that the user uses the absorbent article during night, the selection unit 43 may select the absorbent article for night use. Still furthermore, when the time information indicates that the user uses the absorbent article during night, the selection unit 43 may select the absorbent article for night use, among the absorbent articles according to the physique of the user, instead of the size of underwear that fits the user, and the like.

The generation unit 44 generates content to recommend the selected absorbent article to the user, according to the usage mode (step S106). For example, the generation unit 44 generates suggestion information that recommends the user to use the absorbent article selected by the selection unit 43. Moreover, the generation unit 44 may generate suggestion information indicating the correct usage of the selected absorbent article. Furthermore, the generation unit 44 may generate suggestion information indicating a set of underwear and absorbent article to be recommended to the user.

Still furthermore, when the selected absorbent article matches with the absorbent article indicated in the article information, the generation unit 44 may generate suggestion information that suggests the user to continue using the current absorbent article. Still furthermore, when the selected absorbent article does not match with the absorbent article indicated in the article information, the generation unit 44 may generate suggestion information that suggests the user to switch to the selected absorbent article. Still furthermore, when the selected absorbent article matches with the absorbent article indicated in the article information, but the usage mode information indicates that there is leakage, the generation unit 44 may generate suggestion information indicating the correct wearing position and the like.

Still furthermore, the generation unit 44 may generate information indicating the timing at which the user is recommended to replace the absorbent article, on the basis of the activity information and the exercise amount information. For example, when the amount of exercise indicated in the exercise amount information exceeds a predetermined threshold, the generation unit 44 may generate suggestion information that suggests the user to replace the absorbent article before the exercise. Still furthermore, the generation unit 44 may also specify the timing of exercise of the user from the activity information, and the like, and by taking the specified timing into consideration, estimate the timing to replace the sanitary product estimated to be suitable, and generate suggestion information indicating the estimated replacement timing.

The providing unit 45 provides the generated content (step S107). For example, the providing unit 45 provides suggestion information to the user, by transmitting the suggestion information generated by the generation unit 44 to the terminal device 100.

### 3. Variation of Process

Hereinafter, variation of the process described above will be explained. It is to be noted that the following output process may be performed individually, or may be performed in any combination.

### 3-1. Destination of Information

In the above description, the information providing device 10 provides information on the absorbent article suitable for the user, to the user who are using the absorbent article. However, the embodiment is not limited thereto.

For example, when the user is a young person, it may be considered that the user does not actively acquire information on the absorbent article. Consequently, the information providing device 10 may also provide suggestion information to a parent of the user who is using the absorbent article. For example, the information providing device 10 may provide suggestion information on the absorbent article selected on the basis of the user information of the user U, to mother of the user U.

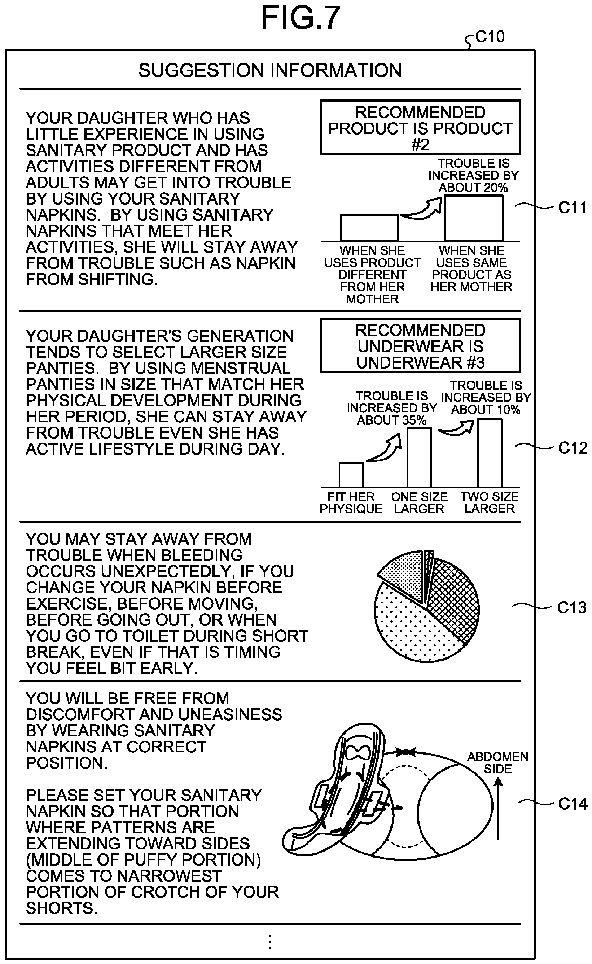

For example, FIG. 7 is a diagram illustrating an example of suggestion information to be suggested to a parent of a user by the information providing device according to the embodiment. For example, the information providing device 10 generates the suggestion information C10 including a content C11 indicating a message suggesting to use a "product #2" that is the absorbent article selected on the basis of the physique information of the user U, an example of trouble caused when the user U uses her parent's absorbent article, an increase in the rate of trouble caused when the user U uses the absorbent article that does not fit the physique of the user U, and the like.

Moreover, the information providing device 10 generates the suggestion information C10 including a content C12 indicating a message suggesting to use an "underwear #3" that is the underwear selected on the basis of the physique information of the user U, an example of trouble caused when the user U uses the underwear that does not fit the physique of the user U, an increase in the rate of trouble caused when the user U uses the underwear that does not fit the physique of the user U, and the like.

Furthermore, the information providing device 10 generates the suggestion information C10 including a content C13 indicating a message explaining an effective replacement timing of the absorbent article to prevent leakage, and a graph indicating the rate of trouble caused when the absorbent article is replaced at a suitable replacement timing, the rate of trouble caused when the absorbent article is not replaced at a suitable replacement timing, and the like.

Still furthermore, the information providing device 10 generates the suggestion information C10 including a content C14 indicating the wearing position and the way of wearing the absorbent article effective to prevent leakage. Then, the information providing device 10 may provide the suggestion information C10 to the user U, or to the parent of the user U. When such suggestion information C10 is provided to the parent of the user U, an appropriate advice can be given to the user U from the parent who is most likely familiar with menstruation than the user U. Thus, for example, it is possible to improve communication between the parent and the child during a rebellious period and the like.

### 3-2. Selection of Absorbent Article

In the above description, the information providing device 10 has selected the absorbent article on the basis of various types of user information. In this example, the information providing device 10 may also select the absorbent article suitable for the user in any combination, in addition to the various combinations described above. For example, the information providing device 10 may provide the absorbent article that hardly shifts, wrinkles, or leaks, as the suitable absorbent article. The information providing device 10 may also provide the absorbent article that is convenient, that can be easily handled, or that is inexpensive. Moreover, the absorbent article is not limited to what is called a sanitary napkin with wings, and may also be a product such as a panty liner, a tampon, a shorts-type absorbent article, or a menstrual cup capable of receiving menstrual blood. The shape, the material, or the way of using the absorbent article is not particularly limited. The characteristics of the product serving as the absorbent article differ depending on the shape and material. For example, the shorts-type absorbent article is convenient because the user only needs to wear the product. However, because the size of one piece of the shorts-type absorbent article is large, it is bulky. The sanitary napkin and the panty liners can be stored in a compact manner, but need to be correctly fitted to the panties. Because it is difficult to select the suitable absorbent article with respect to the user's wish, schedule, lifestyle, and environment, while taking into account such characteristics, it is advantageous for the users to have the information providing device 10 provide information on the suitable absorbent article.

Moreover, the information providing device 10 may select the absorbent article suitable for the user, on the basis of the information on the menstruation management service provided to the user. For example, when the presence of leakage is registered in the menstruation management service, the information providing device 10 may estimate whether the absorbent article currently used by the user U is a suitable absorbent article, from such information.

Moreover, the information providing device 10 may also estimate the menstrual period of the user via the menstruation management service, and select the absorbent article having a function according to the estimated menstrual period. For example, the information providing device 10 acquires the previous menstrual period from the menstruation management service, and estimates the next menstrual period from the acquired menstrual period. Alternatively, the information providing device 10 specifies when the current menstrual period has started from the menstruation management service. Moreover, the information providing device 10 estimates the average menstrual period of the user U.

In this example, the menstrual blood loss tends to increase from the early stage to the middle stage of the menstrual period, and tends to decrease from the middle stage to the final stage. Consequently, for example, when the suggestion information is to be provided before the menstrual period, or at the early or final stage of the menstrual period, the information providing device 10 may select the absorbent article that does not absorb much, but that is thin and that has relatively comfortable wearing feel, as a suggestion target. When the suggestion information is to be provided during the middle stage of the menstrual period, the information providing device 10 may select the absorbent article that absorbs a large amount, as a suggestion target.

Moreover, for example, the information providing device 10 may also suggest a panty liner for discharge, in addition to the absorbent article for menstrual bleeding such as a sanitary napkin. For example, from the various types of information obtained through the menstruation management service, the information providing device 10 may estimate a period during which the user has discharge, and select the panty liner having a size corresponding to the physique of the user and the like, as a suggestion target, when the estimated period has approached.

### 3-3. Stock Control Function

Moreover, the information providing device 10 may estimate the number of stocks of the absorbent article owned by the user, and suggest the user with information according to the estimated number of stocks. For example, the information providing device 10 specifies the number and content of the absorbent article purchased by the user, from the article information. Then, on the basis of the date when the article information is registered, and the information obtained from the menstruation management service, the information providing device 10 estimates the number of stocks of the absorbent articles owned by the user. Then, at the timing when the estimated number of stocks falls below a predetermined threshold, the information providing device 10 may suggest the absorbent article having a size according to the physique of the user, or provide suggestion information that suggests the user to purchase absorbent articles.

Furthermore, for example, to more suitably manage the number of stocks, it is preferable to allow the user to purchase the article provided by the information providing device 10 through the information providing device 10 or the other purchasing service (electronic commerce (EC) site and the like). Consequently, the information providing device 10 can suggest the user to purchase the article, by managing the number of stocks owned by the user, on the basis of information on the number of absorbent articles purchased by the user, the purchased date, the menstrual period, and the like.

### 3-4. Others

Among the processes described above, all or a part of the processes described as being automatically performed may be manually performed. Alternatively, all or a part of the processes described as being manually performed may be automatically performed by a known method. In addition, the processing procedures, specific names, and information including various kinds of data and parameters indicated in the above document and drawings can be optionally changed unless otherwise specified. For example, the various kinds of information illustrated in the drawings are not limited to the illustrated information.

Moreover, the illustrated components of the devices are functionally conceptual, and need not necessarily be physically configured as illustrated. In other words, the specific mode of dispersion and integration of the devices is not limited to the ones illustrated in the drawings. Moreover, all or a part of the components may be functionally or physically dispersed or integrated in an optional unit, depending on various kinds of load and the status of use. Furthermore, the processes described above may be appropriately combined within a range without contradiction.

### 4. Hardware Configuration

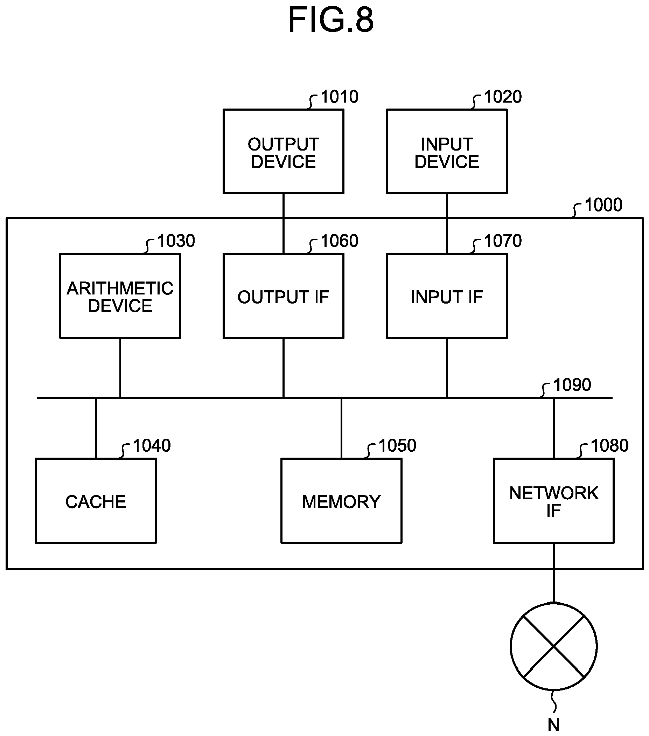

Moreover, for example, the information providing device 10 according to the embodiment described above is implemented by a computer 1000 having a configuration as illustrated in FIG. 8. FIG. 8 is a diagram illustrating an example of a hardware configuration. The computer 1000 is connected to an output device 1010 and an input device 1020. In the computer 1000, an arithmetic device 1030, a cache 1040, a memory 1050, an output interface (IF) 1060, an input IF 1070, and a network IF 1080 are connected via a bus 1090.

The arithmetic device 1030 operates on the basis of a computer program stored in the cache 1040 and the memory 1050, a computer program read out from the input device 1020, or the like, and executes various processes. The cache 1040 is a cache that temporarily stores therein data used for various operations performed by the arithmetic device 1030. Moreover, the memory 1050 is a storage device in which data used for various operations performed by the arithmetic device 1030, and various databases are stored. The memory 1050 is also a memory implemented by a read only memory (ROM), a hard disk drive (HDD), a flash memory, and the like.

The output IF 1060 is an interface used for transmitting information to be output, to the output device 1010 that outputs various types of information such as a monitor and a printer. For example, the output IF 1060 may be implemented by a connector having a standard such as universal serial bus (USB), digital visual interface (DVI), or high definition multimedia interface (HDMI)(registered trademark). Alternatively, the input IF 1070 is an interface used for receiving information from various input devices 1020 such as a mouse, a keyboard, and a scanner. For example, the input IF 1070 is implemented by the USB and the like.

For example, the input device 1020 may be implemented by an optical recording medium such as a compact disc (CD), a digital versatile disc (DVD), and a phase change rewritable disk (PD); a magneto-optical recording medium such as a magneto-optical disk (MO); a tape medium; a magnetic recording medium; or a device that reads out information from a semiconductor memory and the like. Moreover, the input device 1020 may be implemented by an external storage medium such as an USB memory.

The network IF 1080 receives data from the other device via the network N, and sends the data to the arithmetic device 1030. Moreover, the network IF 1080 transmits data generated by the arithmetic device 1030 to the other device via the network N.

In this example, the arithmetic device 1030 controls the output device 1010 and the input device 1020, via the output IF 1060 and the input IF 1070. For example, the arithmetic device 1030 loads a computer program from the input device 1020 or the memory 1050 on the cache 1040, and executes the loaded computer program. For example, when the computer 1000 functions as the information providing device 10, the arithmetic device 1030 of the computer 1000 implements the function of the control unit 40 by executing the computer program loaded on the cache 1040.

As a providing method of the present embodiment, the present embodiment may be provided to the user, by installing a dedicated application in the terminal device 100, and by having the computer program installed in the information providing device 10 and the application installed in the terminal device 100 cooperate with each other. For example, the application in the terminal device 100 causes the terminal device 100 to execute a process of acquiring the article information on the absorbent article used by the user, a display process of an input screen used to input the user information, a reception process for receiving input, a transmission process for transmitting the article information and the user information to the information providing device 10, and the like. Alternatively, the computer program in the information providing device 10 causes the information providing device 10 to execute an acquisition process for acquiring the article information and the user information from the terminal device 100, and a selection process for selecting the absorbent article suitable for the user from the user information.

Moreover, the terminal device 100 may provide the present embodiment to the user, by installing a general browser, and being connected to the information providing device 10 via a general browser of the terminal device 100. For example, the information providing device 10 may implement the process described above, by distributing web content for receiving input of the user information and web content for acquiring the article information, to the terminal device 100, and acquiring the user information and the article information via the web content.

### 5. Advantage

As described above, the information providing device 10 selects the absorbent article suitable for the user, on the basis of the information indicating the physical characteristics of the user, and the preference or the amount of exercise of the user. For example, the information providing device 10 selects the absorbent article the size of which matches the user, on the basis of the physical characteristics of the user. Moreover, the information providing device 10 selects the absorbent article that may motivate the user to use the absorbent article, on the basis of the preference of the user. Furthermore, the information providing device 10 selects the absorbent article having a function to prevent the absorbent article from wrinkling, shifting, and leaking, according to the amount of exercise of the user.

As a result of such a process, the information providing device 10 can suggest the user to use the absorbent article that fits the physique and that matches the amount of exercise of the user. Consequently, the information providing device 10 can reduce the possibility of leakage of secretions, and improve the quality of life of the user. Moreover, the information providing device 10 can motivate the young user who is not familiar with the absorbent article, to use the absorbent article suitable for the user. Consequently, the information providing device 10 can reduce the possibility of leakage of secretions, and improve the quality of life of the user.

The embodiments of the present application have been described in detail with reference to the accompanying drawings. However, the described embodiments are merely examples, and the embodiments of the present application can be carried out in another mode subjected to various modifications and improvements based on the knowledge of those who are skilled in the art, in addition to the modes described in the section of the disclosure of the invention. Moreover, the "section, module, and unit" described above can be read as "means", a "circuit", and the like.

According to one aspect of the embodiment, it is possible to select the absorbent article suitable for the user.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

## Claims

1. A selection device (10), comprising:
an acquisition unit (41) that acquires user information indicating a physical characteristic of a user, and that acquires preference information indicating a preference of the user or exercise amount information indicating an amount of exercise of the user; and
a selection unit (43) that selects an absorbent article to be suggested to the user, based on the user information, and the preference information or the exercise amount information.

2. The selection device (10) according to claim 1, wherein the acquisition unit (41) acquires one or more of; preference information indicating a preference of the user for the absorbent article, for example a preference of the user for appearance of the absorbent article, a preference of the user for a function of the absorbent article, or a preference of the user for an activity corresponding to the function of the absorbent article.

3. The selection device (10) according to any one of claims 1 to 2, wherein the acquisition unit (41) acquires information indicating physique of the user, as the user information, and/or wherein the acquisition unit (41) acquires information indicating age of the user, as the user information.

4. The selection device (10) according to any one of claims 1 to 3, wherein
the acquisition unit (41) further acquires time information indicating time when the user wears the absorbent article, and
the selection unit (43) selects the absorbent article to be suggested to the user, based on the user information, and the preference information, the exercise amount information, or the time information.

5. The selection device (10) according to any one of claims 1 to 4, further comprising:
an estimation unit (42) that estimates circumference of buttocks of the user, based on the user information, wherein
the selection unit (43) selects the absorbent article to be suggested to the user, based on the circumference of the buttocks estimated by the estimation unit (42), and the preference information or the exercise amount information, or wherein;
the acquisition unit (41) acquires information indicating circumference of buttocks of the user, as the user information, and
the selection unit (43) selects the absorbent article to be suggested to the user, based on the circumference of the buttocks of the user, and the preference information or the exercise amount information.

6. The selection device (10) according to claim 5, wherein the selection unit (43) estimates underwear to be recommended to the user, based on the circumference of the buttocks, and selects the absorbent article to be suggested to the user, based on the underwear being estimated.

7. The selection device (10) according to claim 6, wherein the selection unit (43) selects the absorbent article corresponding to a function of the underwear.

8. The selection device (10) according to any one of claims 1 to 7, wherein the selection unit (43) estimates a size of the absorbent article to be recommended, based on the user information, and selects the absorbent article having the size being estimated, and/or wherein the selection unit (43) estimates a function of the absorbent article to be recommended, based on the exercise amount information, and selects the absorbent article having the function being estimated.

9. The selection device (10) according to any one of claims 1 to 8, wherein
the acquisition unit (41) further acquires usage information indicating an absorbent article used by the user and a usage result of the absorbent article, and
the selection unit (43) selects the absorbent article to be suggested to the user, based on the user information, the usage information, and the preference information or the exercise amount information.

10. The selection device (10) according to claim 9, wherein when the usage information indicates that the absorbent article has not functioned properly, the selection unit (43) selects the absorbent article to be suggested to the user, from a plurality of the absorbent articles excluding the absorbent article indicated in the usage information, based on the user information, and the preference information or the exercise amount information.

11. The selection device (10) according to claim 9 or 10, further comprising a first providing unit (45) that provides the user with information on a usage mode of the absorbent article, when the absorbent article selected by the selection unit (43) and the absorbent article indicated in the usage information satisfy a predetermined condition, and when the usage information indicates that the absorbent article has not functioned properly.

12. The selection device (10) according to any one of claims 1 to 11, further comprising a second providing unit (45) that provides the user with information indicating a timing when replacement of the absorbent article estimated based on the exercise amount information is recommended.

13. The selection device (10) according to any one of claims 1 to 12, further comprising a third providing unit (45) that provides the user or another user who has a predetermined relation with the user, with information on the absorbent article selected by the selection unit (43).

14. A selection method executed by a selection device (10), the selection method comprising:
acquiring user information indicating a physical characteristic of a user, and preference information indicating a preference of the user or exercise amount information indicating an amount of exercise of the user; and
selecting an absorbent article to be suggested to the user, based on the user information, and the preference information or the exercise amount information.

15. A non-transitory computer-readable recording medium having stored therein a selection program that causes a computer to execute a process comprising:
acquiring user information indicating a physical characteristic of a user, and preference information indicating a preference of the user or exercise amount information indicating an amount of exercise of the user; and
selecting an absorbent article to be suggested to the user, based on the user information, and the preference information or the exercise amount information.
